# EUROPEAN PATENT APPLICATION

(11) **EP 4 537 874 A1**
(43) Date of publication of application: **16.04.2025**
(21) Application number: 23871273.1
(22) Date of filing: 20.04.2023
(51) Int. Cl.: A61M 5/20, A61M 5/46

(54) **PUNCTURE DEVICE AND DRUG SOLUTION ADMINISTRATING DEVICE PROVIDED WITH PUNCTURE DEVICE**

(30) Priority: 28.09.2022 JP 2022154313
(71) Applicant: Terumo Kabushiki Kaisha, Tokyo 151-0072 (JP)
(72) Inventor: HASEGAWA, Makoto, Ashigarakami-gun, Kanagawa 259-0151 (JP); ABE, Yuichi, Sendai-shi, Miyagi 984-0051 (JP)
(74) Representative: KIPA AB
(86) International application number: PCT/JP2023/015711
(87) International publication number: WO 2024/070024

(57) **Abstract**

An adjustment mechanism (24) of a puncture device (10) includes a plurality of first to third adjustment step portions (166, 168, 170). In a state before skin (S) is punctured by a puncture needle (22), the first to third adjustment step portions (166, 168, 170) have different distances from an abutting portion (104a, 104b) of the needle member (82) in the puncture direction. When the skin (S) is punctured by the puncture needle (22), the abutting portion (104a, 104b) abuts on one of the first to third adjustment step portions (166, 168, 170) according to a position of the adjustment mechanism (24) with respect to a housing (14), whereby a puncture depth of the puncture needle (22) with respect to the skin (S) is adjusted.

## Description

### Technical Field

The present invention relates to a puncture device including a needle member that punctures skin of a living body and a liquid medicine administration device including the puncture device.

### Background Art

European Patent No. 3463523 discloses a puncture device that punctures skin of a user (patient) to be administered with a puncture needle. A puncture device includes a needle assembly including a needle, a drive mechanism, and a contact member. The drive mechanism includes a drive plate and a drive member movably screwed with the drive plate. When the drive member moves in an axial direction, the drive member linearly displaces the needle assembly via the contact member, and performs puncture of the skin by the needle of the needle assembly.

### Summary of Invention

In general, when puncture is performed by the puncture device, it is necessary to change a puncture depth of the needle according to the skin thickness (subcutaneous fat) of the patient. In the puncture device of European Patent No. 3463523, it is necessary to control a movement amount of the drive member in the axial direction when the puncture depth of the needle with respect to the skin is changed. However, drive control of the drive member is complicated, and it is difficult to accurately manage the puncture depth by controlling the movement amount of the drive member.

An object of the present invention is to solve the above problems.
(1) A first aspect of the present invention is a puncture device including: a housing having a contact surface that comes into contact with skin of a living body; a needle member that includes a puncture needle and is movable relative to the housing along an axis of the puncture needle, the puncture needle being capable of protruding from the contact surface toward the skin; and an adjustment mechanism that is displaceable with respect to the housing and adjusts a puncture depth of the puncture needle with respect to the skin, wherein the adjustment mechanism includes a movement preventing portion that abuts on an abutting portion of the needle member and prevents movement in a puncture direction when the puncture needle moves toward the skin to perform puncture of the skin, the movement preventing portion includes a plurality of adjustment step portions having different distances from the abutting portion of the needle member in the puncture direction in a state before the puncture of the skin by the puncture needle is performed, and the abutting portion abuts on one of the plurality of adjustment step portions according to a position of the adjustment mechanism with respect to the housing to adjust a depth of the puncture needle with respect to the skin when the puncture needle moves toward the skin to perform the puncture of the skin.

According to this puncture device, a user operates the adjustment mechanism to set positions of the adjustment step portions with respect to the housing before puncturing the skin with the puncture needle, so that the abutting portion of the needle member can abut on one of the plurality of adjustment step portions according to the positions of the plurality of adjustment step portions with respect to the housing, and the puncture depth of the puncture needle with respect to the skin can be adjusted. Therefore, it is possible to easily select the puncture depth of the puncture needle with respect to the skin of the living body by the adjustment mechanism and to perform the puncture of the skin at a desired depth.

(2) In the puncture device according to (1), the adjustment mechanism may be displaceable along a plane perpendicular to the axis with respect to the housing.

With this configuration, any one of the plurality of adjustment step portions and the abutting portion of the needle member can be disposed to face each other when the puncture of the skin is performed by the puncture needle.

(3) In the puncture device according to (1) or (2), the adjustment mechanism may be rotatable with respect to the housing.

(4) In the puncture device according to any one of (1) to (3), the adjustment mechanism may be rotatable around the axis of the puncture needle with respect to the housing.

With this configuration, the needle member having the puncture needle and the adjustment step portions of the adjustment mechanism can be coaxially disposed, so that the abutting portion of the needle member and the adjustment step portions (movement preventing portion) can reliably abut on each other at the time of puncturing the skin with the puncture needle.

(5) In the puncture device according to any one of (1) to (4), the adjustment mechanism may include an operation portion that is movable along a direction in which the plurality of adjustment step portions are arranged in parallel and protrudes to the outside of the housing.

With this configuration, when the user operates the operation portion, the adjustment member can be easily moved with respect to the housing to cause any one of the plurality of adjustment step portions to face the abutting portion of the needle member.

(6) In the puncture device according to any one of (1) to (5), each of the plurality of adjustment step portions includes a step-portion-side engagement portion including a recess or a projection, and the abutting portion includes a hub-side engagement portion including a projection or a recess to be engaged with the recess or the projection of the step-portion-side engagement portion.

With this configuration, when one adjustment step portion of the plurality of adjustment step portions and the abutting portion abut on each other, the step-portion-side engagement portion and the hub-side engagement portion are engaged, so that a position of the adjustment mechanism is maintained and a desired puncture depth is suitably maintained. Therefore, it is possible to prevent the puncture depth from changing due to erroneous abutment between another adjustment step portion of the plurality of adjustment step portions and the abutting portion.

(7) The puncture device according to any one of (1) to (6) may include a biasing member that is provided in the housing and biases the needle member toward the skin.

With this configuration, the skin can be reliably punctured by causing the puncture needle of the needle member to protrude from the contact surface of the housing by the biasing member.

(8) In the puncture device according to (7), a puncture mechanism that causes the needle member to protrude toward the skin with respect to the contact surface of the housing may be included, the puncture mechanism may include a pusher that has a first inclined portion inclined with respect to the puncture direction of the puncture needle and is movable in the puncture direction with respect to the housing, the biasing member, and a second inclined portion that is provided in the needle member, is inclined with respect to the puncture direction, and abuts on the first inclined portion, the needle member may rotate from a first position to a second position around an axis along the puncture direction, via the first inclined portion and the second inclined portion, when the pusher moves in the puncture direction, and the needle member reaching the second position may move in the puncture direction by a biasing force of the biasing member.

(9) The puncture device according to (8) may include a guide member having a pusher guide that guides the pusher along the puncture direction.

With this configuration, the pusher can be stably moved in the puncture direction by the pusher guide when the user pushes the pusher toward the housing.

(10) The puncture device according to (8) or (9) may include a lock portion that engages with the pusher and prevents the pusher from moving in a direction opposite to the puncture direction against the biasing force of the biasing member when the pusher has moved in the puncture direction by a predetermined distance in a range in which the needle member is not rotated.

With this configuration, when the pusher is locked by the lock portion, a resilient force for puncture can be effectively secured in the biasing member, and reliable puncture can be performed.

(11) In the puncture device according to (10), the guide member may include the lock portion.

(12) A second aspect of the present invention is a liquid medicine administration device including: the puncture device according to any one of (1) to (11); and a prefilled syringe that is filled with a liquid medicine, is connected to the needle member of the puncture device, and delivers the liquid medicine to the puncture needle.

According to the present invention, the user operates the adjustment mechanism to set positions of the adjustment step portions with respect to the housing before puncturing the skin with the puncture needle, so that the abutting portion of the needle member can abut on one of the plurality of adjustment step portions according to the positions of the plurality of adjustment step portions with respect to the housing, and the puncture depth of the puncture needle with respect to the skin can be adjusted. Therefore, it is possible to easily select the puncture depth of the puncture needle with respect to the skin of the living body by the adjustment mechanism and to perform the puncture of the skin at a desired depth.

### Brief Description of Drawings

Fig. 1 is a partially-omitted external perspective view of a liquid medicine administration device including a puncture device according to an embodiment of the present invention.
Fig. 2 is a cross-sectional view illustrating the puncture device in Fig. 1.
Fig. 3 is an enlarged plan view of the vicinity of the puncture device of the liquid medicine administration device in Fig. 1.
Fig. 4 is an enlarged front view illustrating the vicinity of an operation portion of an adjustment mechanism in Fig. 1.
Fig. 5 is a cross-sectional view taken along line V-V in Fig. 2.
Fig. 6 is a partially-omitted side view of the puncture device in an initial state before puncture illustrated in Fig. 2.
Fig. 7 is a side view of the puncture device before puncture in Fig. 6 as viewed from another direction.
Fig. 8 is a side view of the puncture device in Fig. 6 illustrating a state in which a guide member is mounted.
Fig. 9 is a cross-sectional view taken along line IX-IX in Fig. 3.
Fig. 10 is an exploded perspective view of the puncture device.
Fig. 11 is a partial cross-sectional side view of the puncture device in Fig. 8 illustrating a state in which a pusher is pressed in a puncture direction.
Fig. 12 is a cross-sectional view of the puncture device in Fig. 9 illustrating a state in which skin is punctured by a puncture needle of a needle member.
Fig. 13 is a partial cross-sectional side view illustrating a state in which the puncture needle is punctured at a second puncture depth.
Fig. 14 is a partial cross-sectional side view illustrating a state in which the puncture needle is punctured at a third puncture depth.
Fig. 15 is a cross-sectional view of the puncture device in Fig. 11 illustrating a state in which the pusher is further pressed in the puncture direction.

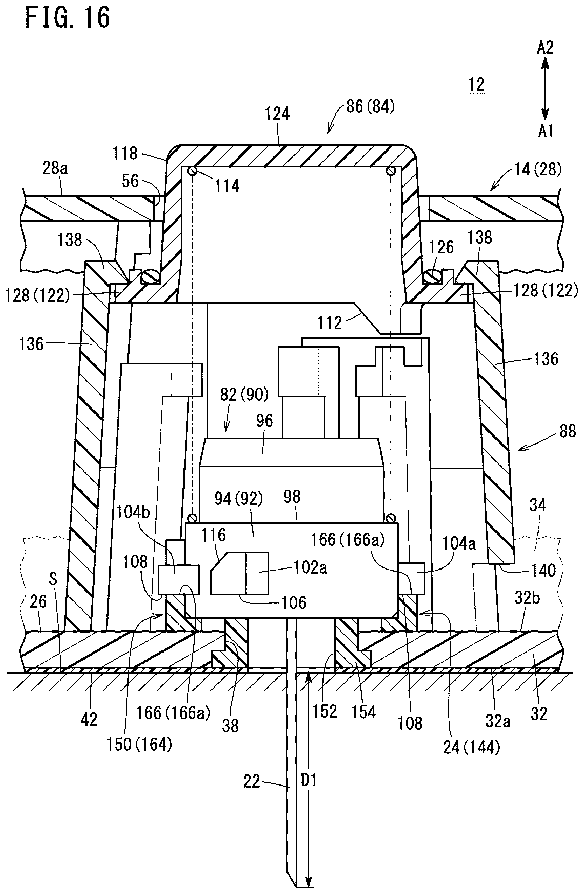

Fig. 16 is a cross-sectional view of the puncture device in Fig. 15 illustrating a puncture state in which the needle member has moved in the puncture direction.
Fig. 17 is a side view of the puncture device at the time of puncture as viewed from another direction.
Fig. 18 is a cross-sectional view taken along line XVIII-XVIII in Fig. 17.

### Description of Embodiments

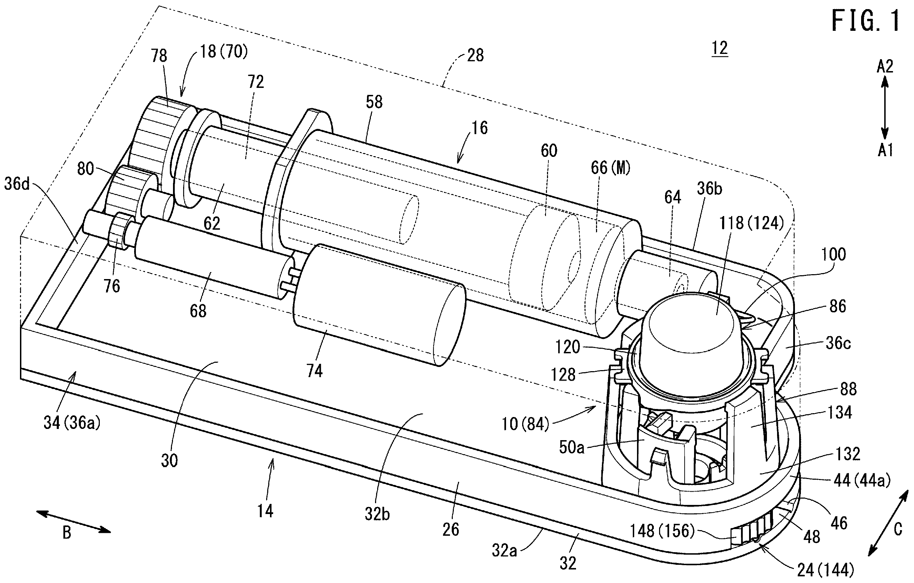

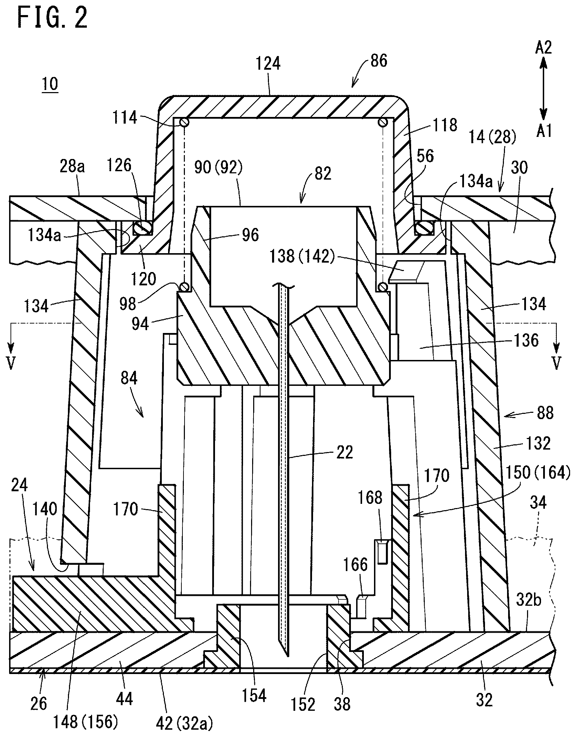

As illustrated in Fig. 1, a puncture device 10 according to the present embodiment is used for a liquid medicine administration device 12 that can administer a liquid medicine M. The liquid medicine administration device 12 is used, for example, to be brought into contact with skin S (refer to Fig. 2) of a patient and administer the liquid medicine M subcutaneously to the patient.

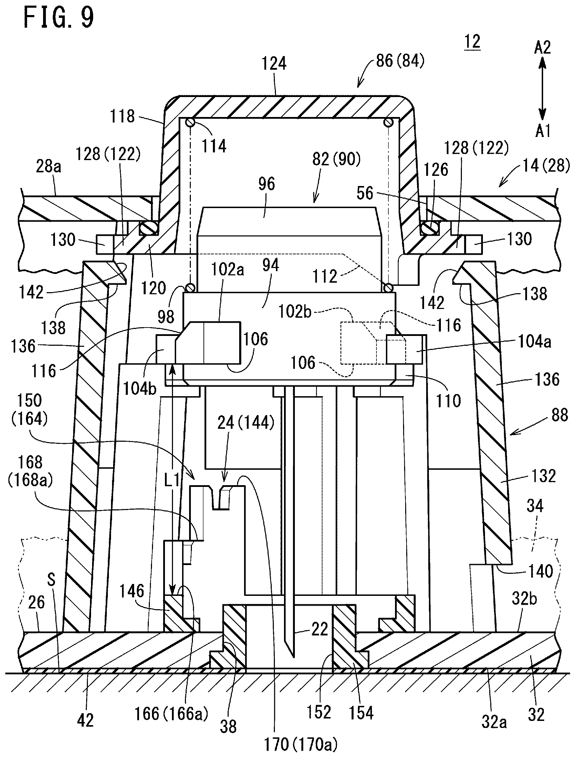

As illustrated in Fig. 1, the liquid medicine administration device 12 includes a housing 14, a prefilled syringe 16 that stores the liquid medicine M, a movement mechanism 18 that delivers the liquid medicine M in the prefilled syringe 16, the puncture device 10 that can puncture the skin S (refer to Fig. 9) of the patient, and an adjustment mechanism 24 that can adjust a puncture depth of a puncture needle 22 (refer to Fig. 9) with respect to the skin S.

The housing 14 serves as both of an enclosure of the liquid medicine administration device 12 and an enclosure of the puncture device 10. That is, the liquid medicine administration device 12 and the puncture device 10 share the housing 14.

The housing 14 includes a housing main body 26 and a housing cover 28. When the liquid medicine administration device 12 including the puncture device 10 administers the liquid medicine M, the housing main body 26 is disposed on the skin S of the patient, and the housing cover 28 is disposed on an opposite side to the skin S with the housing main body 26 therebetween (refer to Fig. 9).

As illustrated in Fig. 2, the housing main body 26 and the housing cover 28 overlap in a thickness direction (arrow A1 and A2 directions) of the housing 14. The arrow A1 direction is a direction toward the skin S when the liquid medicine administration device 12 is installed in the skin S of the patient. The arrow A2 direction is a direction away from the skin S when the liquid medicine administration device 12 is installed in the skin S of the patient. Hereinafter, for convenience of description, a direction from the housing cover 28 toward the housing main body 26 (arrow A1 direction) is referred to as downward, and a direction from the housing main body 26 toward the housing cover 28 (arrow A2 direction) is referred to as upward.

The housing cover 28 covers an upper portion of the housing main body 26. The housing main body 26 and the housing cover 28 can be separated in a vertical direction (arrow A1 and A2 directions in Fig. 1).

As illustrated in Fig. 1, the housing 14 has a space 30 surrounded by the housing main body 26 and the housing cover 28, and the prefilled syringe 16, the movement mechanism 18, and the puncture device 10 are housed in the space 30 of the housing 14.

The housing main body 26 has a bottom wall 32 and a side wall 34 surrounding an outer edge of the bottom wall 32. The side wall 34 is perpendicular to the bottom wall 32. The side wall 34 includes a pair of first width-direction wall portions 36a and second width-direction wall portions 36b, a distal wall portion 36c, and a proximal wall portion 36d. The first width-direction wall portion 36a and the second width-direction wall portion 36b are disposed on both sides in a width direction (arrow C direction) orthogonal to a longitudinal direction (arrow B direction) of the housing main body 26. The first width-direction wall portion 36a and the second width-direction wall portion 36b extend along the longitudinal direction (arrow B direction) of the housing main body 26. The distal wall portion 36c is disposed at a distal portion of the housing main body 26 and is orthogonal to the first width-direction wall portion 36a and the second width-direction wall portion 36b. The proximal wall portion 36d is disposed at a proximal portion of the housing main body 26 and is orthogonal to the first width-direction wall portion 36a and the second width-direction wall portion 36b.

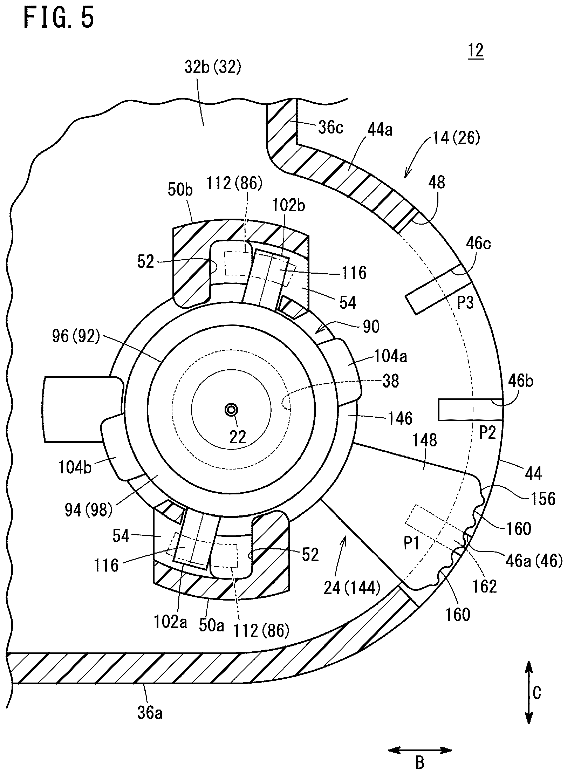

As illustrated in Fig. 2, the bottom wall 32 of the housing main body 26 has a first hole portion 38. The first hole portion 38 is disposed at the distal portion of the housing main body 26 along the longitudinal direction (refer to Fig. 5). The first hole portion 38 passes through the bottom wall 32 and communicates the outside of the housing main body 26 with the space 30.

The bottom wall 32 has a contact surface 32a that comes into contact with the skin S of the patient and a mounting surface 32b on which the adjustment mechanism 24 is disposed. The contact surface 32a is disposed outside the housing main body 26. In the present embodiment, the housing 14 has a bonding material 42 fixed to the bottom wall 32 of the housing main body 26, and one surface of the bonding material 42 is the contact surface. The contact surface 32a is flat along the longitudinal direction (arrow B direction in Fig. 1) of the housing 14 and the width direction (arrow C direction in Fig. 1) orthogonal to the longitudinal direction. The mounting surface 32b is a surface that is disposed inside the housing main body 26 and faces the space 30. The mounting surface 32b is a plane perpendicular to a puncture direction (arrow A1 direction) of the puncture needle 22.

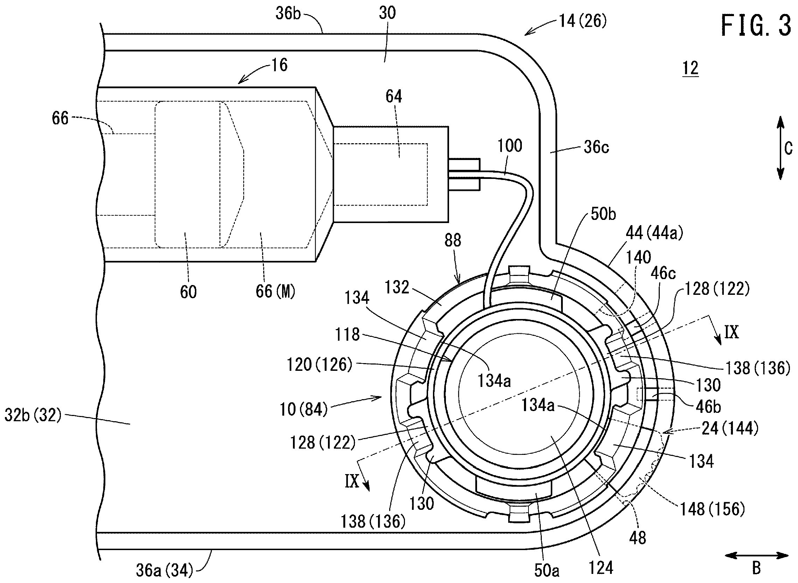

As illustrated in Fig. 3, the bottom wall 32 has an attachment portion 44 which is exposed to the outside and on which the adjustment mechanism 24 is disposed. The attachment portion 44 is disposed in the vicinity of a corner portion between the distal wall portion 36c and the first width-direction wall portion 36a of the housing main body 26. The attachment portion 44 bulges in a substantially semicircular shape from a distal wall of the housing main body 26 toward the distal direction. The mounting surface 32b of the bottom wall 32 is continuous to the attachment portion 44 in the same plane (refer to Fig. 2).

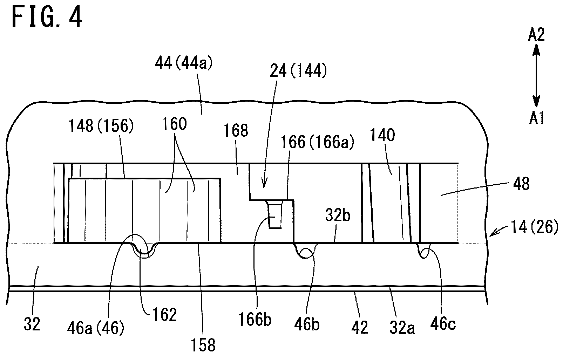

As illustrated in Fig. 4, the mounting surface 32b of the attachment portion 44 includes a plurality of positioning grooves 46. Each of the positioning grooves 46 is recessed from the mounting surface 32b toward the contact surface 32a (arrow A1 direction). The number of the positioning grooves 46 is set to correspond to the number of puncture depths of the puncture needle 22 that can be adjusted by the adjustment mechanism 24. Hereinafter, a case where the positioning grooves 46 include three first to third positioning grooves 46a, 46b, and 46c will be described.

As illustrated in Fig. 5, the first to third positioning grooves 46a, 46b, and 46c extend radially from the center of the first hole portion 38 toward an outer edge portion of the attachment portion 44. The first to third positioning grooves 46a, 46b, and 46c extend in a radial direction in the attachment portion 44. The first to third positioning grooves 46a, 46b, and 46c are spaced at equal intervals in a circumferential direction around the first hole portion 38. The first to third positioning grooves 46a, 46b, and 46c have the same shape.

The first positioning groove 46a is disposed closest to the first width-direction wall portion 36a, and the first positioning groove 46a, the second positioning groove 46b, and the third positioning groove 46c are disposed to be closer to the distal wall portion 36c in this order (refer to Fig. 5).

As illustrated in Fig. 1, a wall portion 44a of the attachment portion 44 has a first opening 48 facing the mounting surface 32b. The first opening 48 is opened in the wall portion 44a, passes through the wall portion 44a, and is formed in an elongated rectangular shape along the mounting surface 32b (refer to Fig. 4). The first opening 48 faces the first to third positioning grooves 46a, 46b, and 46c.

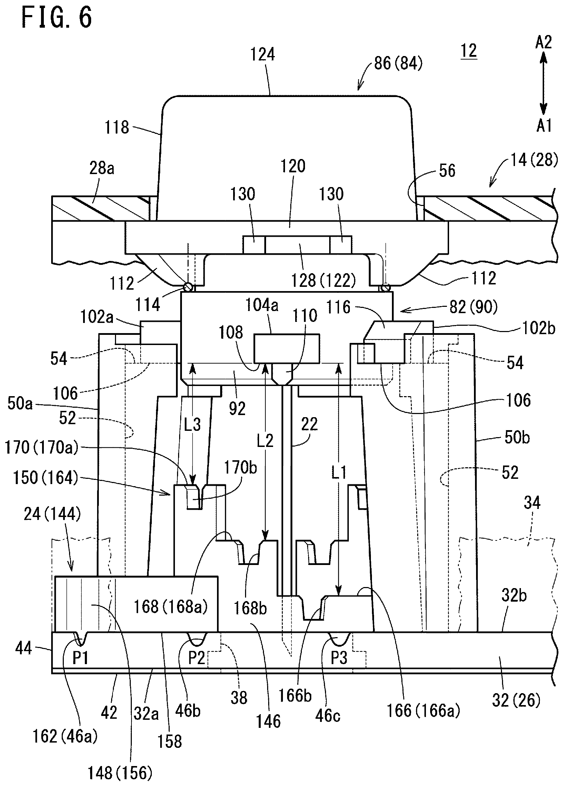

As illustrated in Fig. 6, the housing main body 26 includes a pair of guide columns 50a and 50b. The guide columns 50a and 50b protrude from the bottom wall 32 toward the housing cover 28. The pair of guide columns 50a and 50b faces each other with the first hole portion 38 as the center (refer to Fig. 5). That is, the guide columns 50a and 50b are spaced apart from each other radially outward from the first hole portion 38.

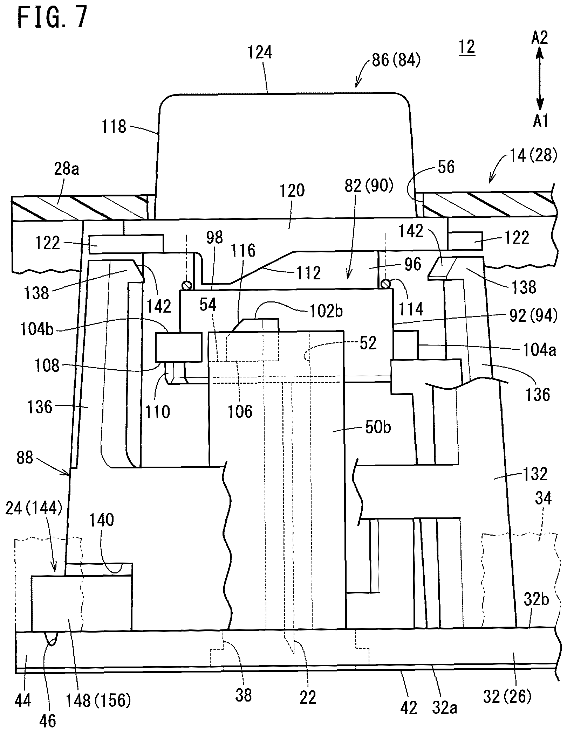

As illustrated in Figs. 6 and 7, each of the guide columns 50a and 50b includes a guide groove 52 capable of guiding a needle member 82 in an axial direction and a locking portion 54 capable of guiding the needle member 82 in a rotation direction. As illustrated in Fig. 5, the guide groove 52 is recessed radially outward from an inner surface facing an axis of the first hole portion 38. A cross-sectional shape of the guide groove 52 is a rectangular shape as viewed from an extending direction of each of the guide columns 50a and 50b illustrated in Fig. 5. The guide groove 52 extends along the extending direction of the guide columns 50a and 50b (refer to Fig. 6). The guide groove 52 of the guide column 50a on one side and the guide groove 52 of the guide column 50b on the other side face each other.

As illustrated in Fig. 6, the locking portion 54 is adjacent to the guide groove 52 in the circumferential direction around the first hole portion 38. The locking portion 54 is a flat surface orthogonal to the axial direction of each of the guide columns 50a and 50b.

An upper wall 28a of the housing cover 28 is formed in parallel with the bottom wall 32 of the housing main body 26. The upper wall 28a of the housing cover 28 has a second hole portion 56. The second hole portion 56 is disposed at a distal portion of the housing cover 28. The first hole portion 38 and the second hole portion 56 face each other in the thickness direction of the housing 14.

As illustrated in Fig. 1, the prefilled syringe 16 is housed in the housing 14 and is disposed along the longitudinal direction (arrow B direction) of the housing 14. The prefilled syringe 16 includes a barrel 58, a gasket 60, and a rod 62. The barrel 58 has a cylindrical shape along an axial direction, of which a proximal end is opened. A distal end of the barrel 58 has a nozzle 64 that protrudes in the distal direction. The gasket 60 is movably housed in the barrel 58. The gasket 60 is made of an elastic material and is liquid-tightly inserted into the barrel 58. A medicine chamber 66 filled with the liquid medicine M is provided between the distal end of the barrel 58 and the gasket 60.

The rod 62 is formed in a cylindrical shape. A distal portion of the rod 62 is inserted into the barrel 58 and is coupled to a proximal end of the gasket 60. A proximal portion of the rod 62 is disposed outside the barrel 58. The rod 62 is disposed to be movable in the axial direction (the distal direction, a proximal direction, and the arrow B direction) relative to the barrel 58.

The movement mechanism 18 includes a motor 68, a gear portion 70, and a shaft 72. The motor 68 is disposed in parallel with the prefilled syringe 16. The motor 68 is electrically connected to a power supply unit 74. The power supply unit 74 is disposed in a distal direction of the motor 68. The power supply unit 74 can supply power to the motor 68. The power supply unit 74 is, for example, a battery. The power is supplied from the power supply unit 74 to the motor 68, and the power rotationally drives a driving shaft (not illustrated) of the motor 68. The motor 68 is driven and controlled by a controller (not illustrated).

The gear portion 70 is disposed in a proximal direction of the motor 68. The gear portion 70 includes a drive gear 76 coupled to the driving shaft, a driven gear 78 coupled to the shaft 72, and an intermediate gear 80 meshed with the drive gear 76 and the driven gear 78. The intermediate gear 80 is disposed between the drive gear 76 and the driven gear 78. The drive gear 76, the driven gear 78, and the intermediate gear 80 are arranged in parallel in the width direction (arrow C direction) of the housing 14.

By rotationally driving the motor 68, the drive gear 76 rotates together with the driving shaft (not illustrated) of the motor 68. As the drive gear 76 rotates, the intermediate gear 80 and the driven gear 78 rotate so that the shaft 72 rotates.

The shaft 72 extends along the axial direction and is inserted into the rod 62. In the rod 62, an outer peripheral surface of the shaft 72 is screwed with an inner peripheral surface of the rod 62. The shaft 72 is housed in the barrel 58 of the prefilled syringe 16 together with the rod 62. The driven gear 78 is coupled to a proximal end of the shaft 72.

When a driving force of the motor 68 is transmitted to the shaft 72 via the gear portion 70 to rotate the shaft 72, the rod 62 screwed with the shaft 72 moves along the axial direction (arrow B direction). The movement of the rod 62 moves the gasket 60 in the barrel 58 along the axial direction together with the rod 62, and the liquid medicine M in the medicine chamber 66 is delivered in the distal direction by the gasket 60.

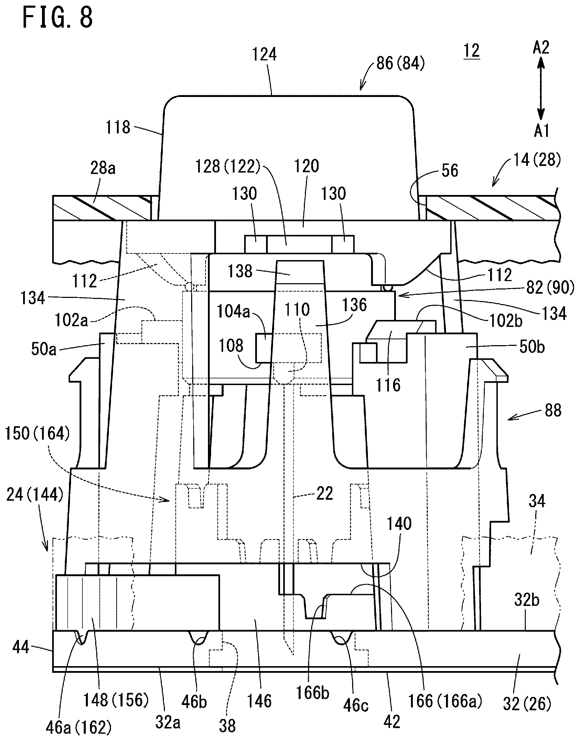

As illustrated in Fig. 3, the puncture device 10 is disposed at a distal portion of the housing 14. The puncture device 10 faces the first and second hole portions 38 and 56 (refer to Fig. 2). As illustrated in Fig. 8, the puncture device 10 includes the needle member 82, a puncture mechanism 84 that biases the needle member 82 toward the skin S of the patient (refer to Fig. 9), and a guide member 88 that can guide a pusher 86 of the puncture mechanism 84 along the axial direction.

As illustrated in Fig. 2, the needle member 82 includes the puncture needle 22 and a needle hub 90 that holds the puncture needle 22. The puncture needle 22 is a hollow needle. A distal end of the puncture needle 22 can puncture the skin S of the patient. A proximal end of the puncture needle 22 is held at an axial center of the needle hub 90.

The needle hub 90 includes a hub main body 92. The hub main body 92 has a circular shape as viewed from an axial direction of the needle hub 90 (refer to Fig. 5). The hub main body 92 includes a large diameter portion 94 and a small diameter portion 96. The large diameter portion 94 is disposed in the distal direction (arrow A1 direction) of the hub main body 92. The small diameter portion 96 is disposed in a proximal direction (arrow A2 direction) of the large diameter portion 94. A diameter of the small diameter portion 96 is smaller than a diameter of the large diameter portion 94. An annular receiving portion 98 extending in a radial direction is provided at a boundary between the small diameter portion 96 and the large diameter portion 94.

The large diameter portion 94 of the hub main body 92 includes a communication path (not illustrated). A distal end of the communication path communicates with the inside of the puncture needle 22. The prefilled syringe 16 and a proximal end of the communication path are connected by an elastically deformable tube 100 (refer to Fig. 3). As illustrated in Fig. 3, the tube 100 is a tubular body and is disposed at the distal portion of the housing 14. The tube 100 has flexibility and can deliver the liquid medicine M from the prefilled syringe 16 to the needle member 82. The liquid medicine M is supplied from the prefilled syringe 16 to the communication path of the needle member 82 (not illustrated) through the tube 100, and the liquid medicine M is delivered from the communication path to the puncture needle 22.

As illustrated in Fig. 5, an outer peripheral surface of the needle hub 90 has a pair of guided portions 102a and 102b and a pair of abutting portions 104a and 104b. The pair of guided portions 102a and 102b protrudes radially outward from an outer peripheral surface of the hub main body 92. The guided portion 102a on one side and the guided portion 102b on the other side are symmetrically disposed with respect to an axial center of the hub main body 92. As illustrated in Fig. 6, each of the guided portions 102a and 102b has a locking surface 106. The locking surfaces 106 are disposed at distal ends of the guided portion 102a and 102b, respectively. The locking surface 106 has a flat shape orthogonal to the axial direction of the needle hub 90.

In an initial state before the skin S is punctured by the puncture needle 22 illustrated in Fig. 6, the locking surfaces 106 of the needle hub 90 abut on the locking portions 54 of the guide columns 50a and 50b. As a result, the needle hub 90 is prevented from moving in the puncture direction (the distal direction and the arrow A1 direction) with respect to the housing 14 including the guide columns 50a and 50b.

The pair of abutting portions 104a and 104b protrudes radially outward from the outer peripheral surface of the hub main body 92. Each of the abutting portions 104a and 104b extends along a circumferential direction of the hub main body 92. The abutting portion 104a on one side and the abutting portion 104b on the other side are disposed symmetrically with respect to the axial center of the hub main body 92. The abutting portions 104a and 104b and the guided portions 102a and 102b are spaced apart from each other in the circumferential direction of the hub main body 92. As illustrated in Fig. 6, each of the abutting portions 104a and 104b has an abutting surface 108 and a hub-side engagement portion 110 protruding from the abutting surface 108. The abutting surfaces 108 are disposed on end surfaces of the abutting portions 104a and 104b in the puncture direction of the needle member 82. The abutting surfaces 108 are flat surfaces orthogonal to the axial direction of the hub main body 92.

The hub-side engagement portion 110 protrudes from the abutting surface 108 in the axial direction of the hub main body 92. A protruding direction of the hub-side engagement portion 110 is the puncture direction (arrow A1 direction) of the needle member 82. A distal end of the hub-side engagement portion 110 gradually tapers toward the distal end.

The puncture mechanism 84 causes the puncture needle 22 of the needle member 82 to protrude from the contact surface 32a of the housing 14 in accordance with an operation of a user (for example, the patient or a medical personnel). The puncture mechanism 84 includes the pusher 86 in which a pair of first inclined portions 112 is provided, a biasing member 114, and a pair of second inclined portions 116 provided on the needle hub 90 and abutting on the first inclined portions 112.

The pusher 86 is disposed to be movable in the puncture direction (the axial direction and the arrow A1 direction) of the puncture needle 22 with respect to the housing 14. The pusher 86 is disposed in the proximal direction (arrow A2 direction) of the needle member 82. Before the skin S is punctured by the puncture needle 22 illustrated in Fig. 2, a part of the small diameter portion 96 of the needle hub 90 is inserted into the pusher 86.

As illustrated in Fig. 9, the pusher 86 includes a pressing portion 118, a flange portion 120, and a pair of protruding portions 122. The pressing portion 118 has a bottomed cylindrical shape and is inserted into the second hole portion 56 of the housing cover 28. The pressing portion 118 is exposed to the outside of the housing 14 through the second hole portion 56. A top of the pressing portion 118 has a pressing surface 124 orthogonal to the axial direction of the pusher 86. When the skin S is punctured by the puncture device 10, the user presses the pressing surface 124 of the pusher 86 toward the housing 14 (puncture direction).

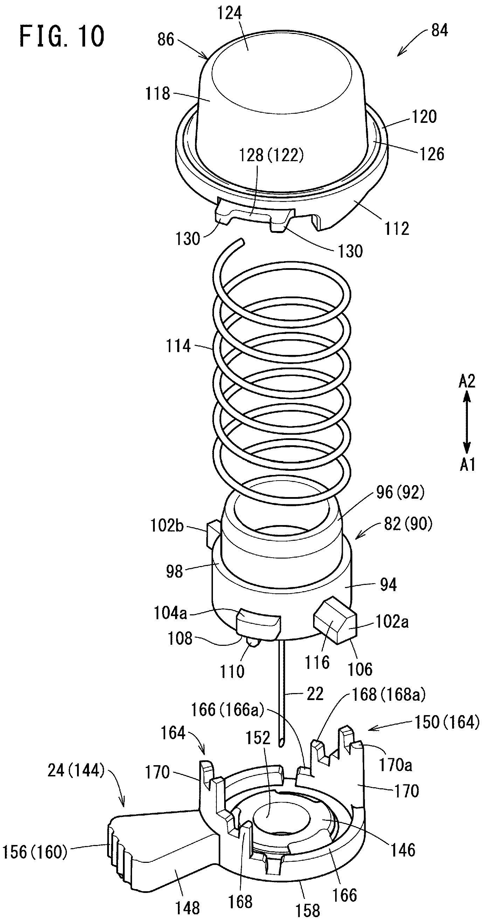

The flange portion 120 is disposed at an end of the pusher 86 on a side closer to the needle hub 90. As illustrated in Fig. 10, the flange portion 120 is formed in an annular shape and is disposed at an outer edge portion of the pressing portion 118. The flange portion 120 has a flange shape protruding radially outward from an outer peripheral surface of the pressing portion 118. As illustrated in Fig. 9, the flange portion 120 is disposed inside the housing 14. The flange portion 120 is disposed radially outward of the second hole portion 56. The flange portion 120 faces an inner surface of the housing cover 28. A diameter of the flange portion 120 is larger than a diameter of the second hole portion 56.

The flange portion 120 has an annular groove adjacent to the outer peripheral surface of the pressing portion 118, and a seal ring 126 is attached to the annular groove. The seal ring 126 is disposed to face the inner surface of the housing cover 28 so as to be contactable with the inner surface.

As illustrated in Fig. 3, the pair of protruding portions 122 protrudes radially outward from an outer peripheral surface of the flange portion 120. The protruding portion 122 on one side and the protruding portion 122 on the other side are disposed symmetrically with respect to an axial center of the pusher 86. Each of the protruding portions 122 has a pusher-side engagement portion 128 and a pair of guide pieces 130. The pair of guide pieces 130 is disposed at both ends of the pusher-side engagement portion 128 in an extending direction, and further protrudes radially outward from the pusher-side engagement portion 128. The pusher-side engagement portion 128 is disposed between the pair of guide pieces 130.

As illustrated in Fig. 10, the biasing member 114 is a spring member including a coil spring. As illustrated in Fig. 2, the biasing member 114 is disposed between the inside of the pressing portion 118 of the pusher 86 and the receiving portion 98 of the needle hub 90. A biasing force of the biasing member 114 is applied to the needle hub 90 and the pusher 86. The biasing member 114 biases the needle member 82 toward the skin S of the patient. As the needle member 82 is biased toward the skin S of the patient by the biasing member 114, the needle member 82 can move in the puncture direction (arrow A1 direction) relative to the housing 14.

As illustrated in Fig. 6, the pair of first inclined portions 112 is disposed in the flange portion 120 of the pusher 86. The first inclined portions 112 protrude from the flange portion 120 toward the bottom wall 32 of the housing main body 26. The pair of first inclined portions 112 is disposed symmetrically with respect to the axial center of the pusher 86. The respective first inclined portions 112 are inclined with respect to the axial direction of the pusher 86. In other words, the first inclined portions 112 are inclined with respect to the puncture direction (arrow A1 direction) of the puncture needle 22. When the pusher 86 is viewed in the puncture direction, the first inclined portions 112 are inclined so as to extend clockwise in the puncture direction (arrow A1 direction).

As illustrated in Fig. 5, the pair of second inclined portions 116 is disposed on the respective guided portions 102a and 102b of the needle hub 90. The second inclined portions 116 are disposed at proximal ends of the guided portions 102a and 102b (refer to Fig. 6). The first inclined portions 112 of the pusher 86 can abut on the second inclined portions 116. The second inclined portions 116 are inclined with respect to the puncture direction (arrow A1 direction) of the needle member 82. That is, the second inclined portions 116 are inclined so as to extend clockwise in the distal direction (arrow A1 direction) when the needle member 82 is viewed from the proximal end in the distal direction. The second inclined portions 116 are inclined in the same direction as the first inclined portions 112.

As illustrated in Fig. 8, the guide member 88 can guide the pusher 86 along the axial direction when the puncture of the skin S is performed by the puncture needle 22. The guide member 88 is housed in the housing 14 (refer to Fig. 3). The guide member 88 is fixed to the housing main body 26 and is erected from the bottom wall 32 toward the housing cover 28. The guide member 88 partially surrounds the pusher 86 and the needle member 82.

The guide member 88 has a guide main body 132 having a tubular shape, a pair of pusher guides 134, a pair of elastically deformable portions 136, and a lock portion 138. The guide main body 132 is disposed so as to surround the first hole portion 38 and abuts on the mounting surface 32b of the bottom wall 32 of the housing main body 26. One end (end on a side closer to the bottom wall 32) of the guide main body 132 faces the mounting surface 32b of the housing main body 26.

The one end of the guide main body 132 has a second opening 140. The second opening 140 is opened by cutting out a part of an outer peripheral surface of the guide main body 132. The second opening 140 is formed in a rectangular shape along the outer peripheral surface of the guide main body 132. The second opening 140 faces the first opening 48 of the housing main body 26 (refer to Fig. 3). The first opening 48 and the second opening 140 have substantially the same size. The inside of the housing 14 and the inside of the guide member 88 communicate with the outside of the housing 14 through the first and second openings 48 and 140. The second opening 140 is opened toward the mounting surface 32b of the housing 14.

As illustrated in Fig. 2, the pair of pusher guides 134 can guide the pusher 86 along the puncture direction. The pair of pusher guides 134 protrudes from the guide main body 132 toward the housing cover 28. The pair of pusher guides 134 is disposed symmetrically with respect to an axial center of the guide member 88 (refer to Fig. 3). An end of the pusher guide 134 has a guide surface 134a.

The guide surface 134a is disposed on the inner periphery of the end of the pusher guide 134 and faces the outer peripheral surface of the pressing portion 118 of the pusher 86. The guide surface 134a has an arcuate cross section corresponding to the outer peripheral surface of the pressing portion 118 (refer to Fig. 3). There is a gap between the guide surface 134a and the outer peripheral surface of the pusher 86. When the pusher 86 moves along the axial direction, the outer peripheral surface of the pusher 86 is guided along the axial direction (arrow A1 and A2 directions) by the guide surface 134a of the pusher guide 134.

The inner surface of the housing cover 28 abuts on the ends of the pair of pusher guides 134. The housing cover 28 is held by the pusher guides 134.

As illustrated in Fig. 9, the pair of elastically deformable portions 136 extends along the axial direction of the guide main body 132. The elastically deformable portions 136 protrude from the guide main body 132 toward the upper wall 28a of the housing cover 28. Ends (root portions) of the elastically deformable portions 136 on the A1 side are fixed ends supported by the guide main body 132. Ends of the elastically deformable portions 136 on the A2 side are free ends. The elastically deformable portions 136 are elastically deformable in the radial direction with the root portions of the elastically deformable portions 136 as fulcrums. As illustrated in Fig. 3, the pair of elastically deformable portions 136 is disposed symmetrically with respect to the axial center of the guide member 88. The elastically deformable portions 136 and the pusher guides 134 are spaced apart from each other in a circumferential direction of the guide member 88.

As illustrated in Fig. 9, the pair of lock portions 138 is disposed at the free ends of the pair of elastically deformable portions 136. The lock portions 138 protrude radially inward from the free ends of the elastically deformable portions 136, respectively. When the pusher 86 is disposed inside the guide member 88, the lock portions 138 face the outer peripheral surface of the pusher 86 and face the pusher-side engagement portions 128 of the protruding portions 122, respectively.

That is, the respective lock portions 138 protrude from the free ends of the elastically deformable portions 136 toward the outer peripheral surface of the pusher 86. Each of the lock portion 138 has an inclined surface 142 facing the pusher 86. The inclined surface 142 is inclined radially inward in the puncture direction.

In an initial state before puncture of the puncture device 10 illustrated in Fig. 9, radially inner ends of the lock portions 138 toward the pusher 86 are disposed radially inward of radially outer ends of the pusher-side engagement portions 128, respectively. In the initial state of the puncture device 10, the pusher-side engagement portions 128 are disposed on the housing cover 28 side with respect to the lock portions 138, respectively.

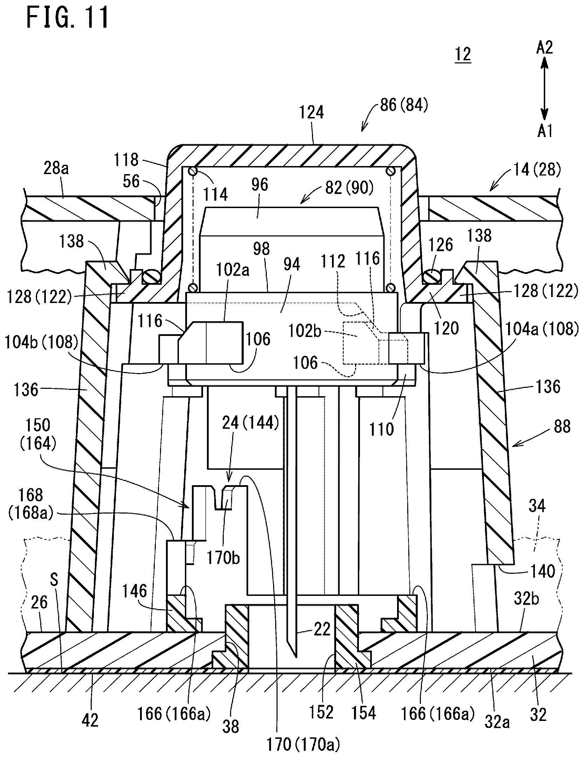

When the pusher 86 is pushed into the housing 14 from the initial state illustrated in Fig. 9, the lock portions 138 are pressed radially outward by the pusher-side engagement portions 128, respectively, and the elastically deformable portions 136 are elastically deformed radially outward. After the pusher-side engagement portions 128 gets over the lock portions 138 as illustrated in Fig. 11, the pusher-side engagement portions 128 are locked by the lock portions 138, respectively. As a result, the pusher 86 having the pusher-side engagement portions 128 is prevented from moving toward the housing cover 28 (arrow A2 direction). In other words, the pusher 86 is prevented from moving in a direction opposite to the puncture direction of the puncture needle 22 is prevented.

As illustrated in Fig. 3, the adjustment mechanism 24 is disposed at the distal portion of the housing 14. The adjustment mechanism 24 includes an adjustment member 144 rotatable along the bottom wall 32 of the housing main body 26. That is, the adjustment member 144 is disposed to be movable in a direction orthogonal to the puncture direction (arrow A1 direction) of the needle member 82 with respect to the housing 14.

As illustrated in Fig. 6, the adjustment member 144 is disposed on the mounting surface 32b of the bottom wall 32 of the housing main body 26. The adjustment member 144 is rotatable along the mounting surface 32b perpendicular to the puncture direction (arrow A1 direction) of the puncture needle 22.

As illustrated in Fig. 10, the adjustment member 144 has a main body portion 146 having a disk shape, an operation portion 148, and a movement preventing portion 150. An insertion hole 152 through which the puncture needle 22 of the needle member 82 can be inserted is provided at the center of the main body portion 146. As illustrated in Fig. 6, the main body portion 146 faces the mounting surface 32b of the housing main body 26. The main body portion 146 has a plurality of fixing portions 154 protruding toward the bottom wall 32 of the housing 14 (refer to Fig. 2).

As illustrated in Fig. 2, the plurality of fixing portions 154 are disposed radially outward of the insertion hole 152 around the insertion hole 152. The plurality of fixing portions 154 are engaged with the first hole portion 38 of the bottom wall 32 of the housing 14. As a result, the adjustment member 144 is rotatably supported with respect to the housing main body 26 via the main body portion 146. The first hole portion 38 of the housing main body 26 and the insertion hole 152 are coaxially disposed to face each other, and the adjustment member 144 is rotatably disposed around the axis of the puncture needle 22 inserted into the first hole portion 38 and the first hole portion 38.

As illustrated in Fig. 5, the operation portion 148 has an operation end 156 that is touched and operated by the user when the puncture depth of the puncture needle 22 is adjusted by the adjustment mechanism 24. The operation portion 148 protrudes radially outward from an outer peripheral surface of the main body portion 146 and extends by a predetermined length in a direction orthogonal to the axis of the main body portion 146. The operation portion 148 has a predetermined thickness in an axial direction of the main body portion 146. An opposing surface 158 of the operation portion 148 is a plane facing the mounting surface 32b of the housing main body 26 and the attachment portion 44 (refer to Fig. 6). When the adjustment member 144 is rotated with respect to the housing 14 by the operation of the user, the operation portion 148 moves along the mounting surface 32b of the housing main body 26 and the attachment portion 44. The operation portion 148 rotates in a substantially arc shape along the mounting surface 32b of the attachment portion 44 around the axis of the main body portion 146 (first hole portion 38) as a rotation center.

The operation end 156 is disposed at an end of the operation portion 148 that is farthest radially outward from the main body portion 146. The operation end 156 is disposed at the outer edge portion of the attachment portion 44. The operation end 156 has a plurality of grooves 160 extending along the axial direction of the main body portion 146 and recessed radially inward. The grooves 160 are arranged in parallel along a moving direction of the operation portion 148. As viewed from the axial direction of the main body portion 146, the operation portion 148 has a substantially fan shape gradually expanding from the main body portion 146 toward the operation end 156.

As illustrated in Fig. 3, the operation portion 148 is inserted into each of the first opening 48 of the housing main body 26 and the second opening 140 of the guide member 88. The operation end 156 of the operation portion 148 is exposed to the outside of the housing 14 through the first opening 48.

As illustrated in Fig. 4, the opposing surface 158 of the operation portion 148 has a protrusion 162. The protrusion 162 protrudes from the opposing surface 158 toward the housing main body 26 and extends along an extending direction of the operation portion 148 (refer to Fig. 5). When the puncture depth of the puncture needle 22 is selected by the adjustment mechanism 24, the protrusion 162 is inserted into and engaged with any one of the first to third positioning grooves 46a, 46b, and 46c. A cross-sectional shape of the protrusion 162 viewed from the extending direction of the operation portion 148 is a semicircular shape. The shape of the protrusion 162 corresponds to the shape of the first to third positioning grooves 46a, 46b, and 46c.

When adjusting the puncture depth of the skin S by the puncture needle 22 by the adjustment mechanism 24, the user moves the adjustment member 144 along the attachment portion 44 by the operation end 156 of the operation portion 148, whereby the adjustment member 144 can be rotated along the mounting surface 32b around the main body portion 146. At this time, the protrusion 162 of the operation portion 148 is engaged with any one of the first to third positioning grooves 46a, 46b, and 46c, whereby the operation portion 148 in the attachment portion 44 is prevented from moving and is positioned at a predetermined position. That is, the rotation of the adjustment member 144 along the mounting surface 32b is restricted.

As illustrated in Fig. 6, when the puncture needle 22 moves toward the skin S to puncture the skin S, the movement preventing portion 150 prevents the movement of the needle member 82 in the puncture direction (arrow A1 direction) by the abutment with the abutting portions 104a and 104b of the needle member 82. The movement preventing portion 150 extends from an outer edge portion of the main body portion 146 along the axial direction of the main body portion 146. A pair of the movement preventing portions 150 is disposed symmetrically with respect to the center of the main body portion 146 (refer to Fig. 10).

Each of the movement preventing portions 150 has a plurality of adjustment step portions 164. In the state before the skin S is punctured by the puncture needle 22, the plurality of adjustment step portions 164 have different spacing distances from the abutting portions 104a and 104b of the needle member 82 in the puncture direction. Hereinafter, a case where the movement preventing portion 150 includes three first to third adjustment step portions 166, 168, and 170 will be described.

As illustrated in Fig. 10, the first to third adjustment step portions 166, 168, and 170 are disposed on the outer edge portion of the main body portion 146. The first to third adjustment step portions 166, 168, and 170 are arranged in parallel in a circumferential direction of the main body portion 146. Along the axial direction of the main body portion 146, when the main body portion 146 is viewed in the puncture direction, the first adjustment step portion 166, the second adjustment step portion 168, and the third adjustment step portion 170 are disposed clockwise in this order.

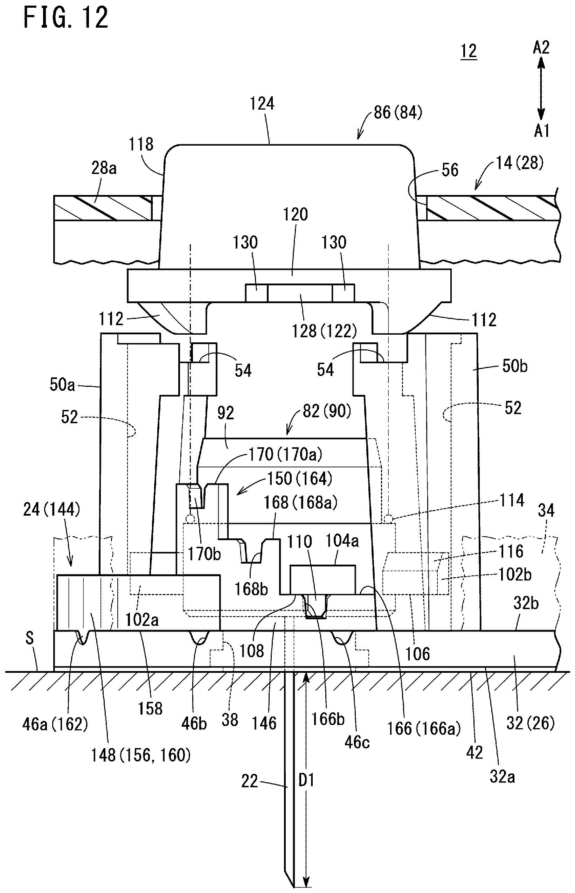

A pair of the first adjustment step portions 166 is disposed symmetrically with respect to the axial center of the main body portion 146. Each of the first adjustment step portions 166 has a first abutted portion 166a on which the abutting portions 104a and 104b of the needle hub 90 can abut. The first abutted portion 166a extends in a direction orthogonal to the axial direction of the main body portion 146. As illustrated in Fig. 12, when the needle hub 90 moves in the puncture direction (arrow A1 direction) at the time of puncturing the skin S with the puncture needle 22, the first abutted portions 166a and the abutting surfaces 108 of the abutting portions 104a and 104b can abut on each other.

As illustrated in Fig. 9, in the initial state before the skin S is punctured by the puncture needle 22, the first abutted portions 166a of the pair of first adjustment step portions 166 and the abutting portions 104a and 104b of the needle member 82 are spaced apart from each other by a first distance L1 in the puncture direction.

As illustrated in Fig. 6, the first abutted portion 166a has a first-step-portion-side engagement portion 166b. The first-step-portion-side engagement portion 166b is recessed in the axial direction of the hub main body 92 from an end surface of the first abutted portion 166a. The first-step-portion-side engagement portion 166b is recessed in the puncture direction (arrow A1 direction) of the needle member 82. The first-step-portion-side engagement portion 166b gradually tapers in the puncture direction.

As illustrated in Fig. 12, when the adjustment member 144 is rotated by the operation portion 148 and the protrusion 162 of the operation portion 148 is engaged with the first positioning groove 46a, the abutting portions 104a and 104b of the needle hub 90 face the first adjustment step portions 166, respectively, at a first adjustment position P1 (refer to Fig. 5). When the needle hub 90 moves in the puncture direction at the first adjustment position P1, the abutting surfaces 108 of the abutting portions 104a and 104b abut on the first abutted portions 166a, respectively. The hub-side engagement portions 110 are engaged with the first-step-portion-side engagement portions 166b, respectively. At this time, the puncture depth of the puncture needle 22 with respect to the skin S becomes a first puncture depth D1. Note that the puncture depth refers to a depth from the skin S of the patient to the distal end of the puncture needle 22 punctured subcutaneously. The first puncture depth D1 is, for example, 12 mm. When the abutting portions 104a and 104b of the needle hub 90 abut on the first abutted portions 166a, respectively, the hub-side engagement portions 110 are engaged with the first-step-portion-side engagement portions 166b, respectively.

As illustrated in Fig. 10, a pair of the second adjustment step portions 168 is disposed symmetrically with respect to the axial center of the main body portion 146. When the movement preventing portion 150 is viewed in the puncture direction of the needle member 82, the second adjustment step portions 168 are adjacent to the first adjustment step portions 166, respectively, clockwise in the circumferential direction of the main body portion 146. As illustrated in Fig. 6, the second adjustment step portion 168 is connected to a circumferential side portion of the first adjustment step portion 166. The second adjustment step portions 168 have second abutted portions 168a which can abut on the abutting portions 104a and 104b, respectively, of the needle hub 90. The second abutted portion 168a extends in a direction orthogonal to the axial direction of the main body portion 146.

As illustrated in Fig. 6, in the initial state before the skin S is punctured by the puncture needle 22, the second abutted portions 168a of the pair of second adjustment step portions 168 and the abutting portions 104a and 104b of the needle member 82 are spaced apart from each other by a second distance L2 in the puncture direction. The second distance L2 is smaller than the first distance L1 (L2 < L1). That is, the second adjustment step portions 168 are disposed closer to the abutting portions 104a and 104b of the needle member 82 than the first adjustment step portions 166. In other words, a protruding height of the second adjustment step portion 168 with respect to the main body portion 146 is larger than a protruding height of the first adjustment step portion 166 with respect to the main body portion 146.

The second abutted portion 168a has a second-step-portion-side engagement portion 168b. The second-step-portion-side engagement portion 168b is recessed in the axial direction of the hub main body 92 from an end surface of the second abutted portion 168a. The second-step-portion-side engagement portion 168b is recessed in the puncture direction (arrow A1 direction) of the needle member 82. The second-step-portion-side engagement portion 168b gradually tapers in the puncture direction.

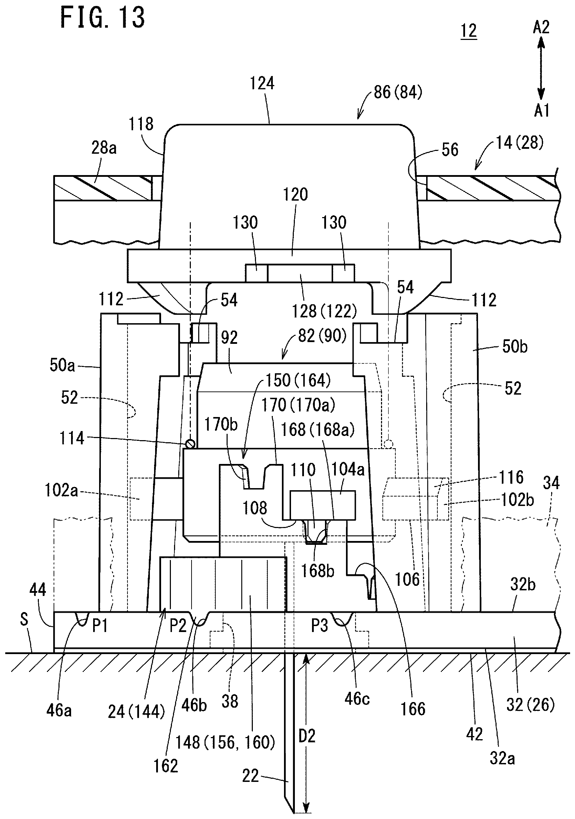

As illustrated in Fig. 13, when the adjustment member 144 is rotated by the operation portion 148 and the protrusion 162 of the operation portion 148 is engaged with the second positioning groove 46b, the abutting portions 104a and 104b of the needle hub 90 face the second adjustment step portions 168, respectively, at a second adjustment position P2. When the needle hub 90 moves in the puncture direction at the second adjustment position P2, the abutting surfaces 108 of the abutting portions 104a and 104b abut on the second abutted portions 168a, respectively. At this time, the puncture depth of the puncture needle 22 with respect to the skin S becomes a second puncture depth D2. The second puncture depth D2 is smaller than the first puncture depth D1, and is, for example, 9 mm. When the abutting portions 104a and 104b of the needle hub 90 abut on the second abutted portions 168a, respectively, the hub-side engagement portions 110 are engaged with the second-step-portion-side engagement portions 168b, respectively. That is, when the second adjustment position P2 is selected by the adjustment mechanism 24, the puncture depth becomes shallower than the first puncture depth D1 at the first adjustment position P1.

As illustrated in Fig. 10, a pair of the third adjustment step portions 170 is disposed symmetrically with respect to the axial center of the main body portion 146. The third adjustment step portion 170 on one side is disposed so as to face the operation portion 148. When the movement preventing portion 150 is viewed in the puncture direction of the needle member 82, the third adjustment step portions 170 are adjacent to the second adjustment step portions 168, respectively, clockwise in the circumferential direction of the main body portion 146. The third adjustment step portion 170 is connected to a circumferential side portion of the second adjustment step portion 168. The third adjustment step portions 170 have third abutted portions 170a facing the abutting portions 104a and 104b, respectively, of the needle hub 90. The third abutted portion 170a extends in a direction orthogonal to the axial direction of the main body portion 146. When the needle hub 90 moves in the puncture direction at the time of puncturing the skin S with the puncture needle 22, the third abutted portions 170a and the abutting surfaces 108 of the abutting portions 104a and 104b can abut on each other.

As illustrated in Fig. 6, in the initial state before the skin S is punctured by the puncture needle 22, the third abutted portions 170a of the pair of third adjustment step portions 170 and the abutting portions 104a and 104b of the needle member 82 are spaced apart from each other by a third distance L3 in the puncture direction. The third distance L3 is smaller than the second distance L2 (L3 < L2 < L1). That is, the third adjustment step portions 170 are disposed closer to the abutting portions 104a and 104b of the needle member 82 than the second adjustment step portions 168. In other words, a protruding height of the third adjustment step portion 170 with respect to the main body portion 146 is larger than the protruding height of the second adjustment step portion 168 with respect to the main body portion 146.

The third abutted portion 170a has a third-step-portion-side engagement portion 170b. The third-step-portion-side engagement portion 170b is recessed in the axial direction of the hub main body 92 from an end surface of the third abutted portion 170a. The third-step-portion-side engagement portion 170b is recessed in the puncture direction (arrow A1 direction) of the needle member 82. The third-step-portion-side engagement portion 170b gradually tapers in the puncture direction.

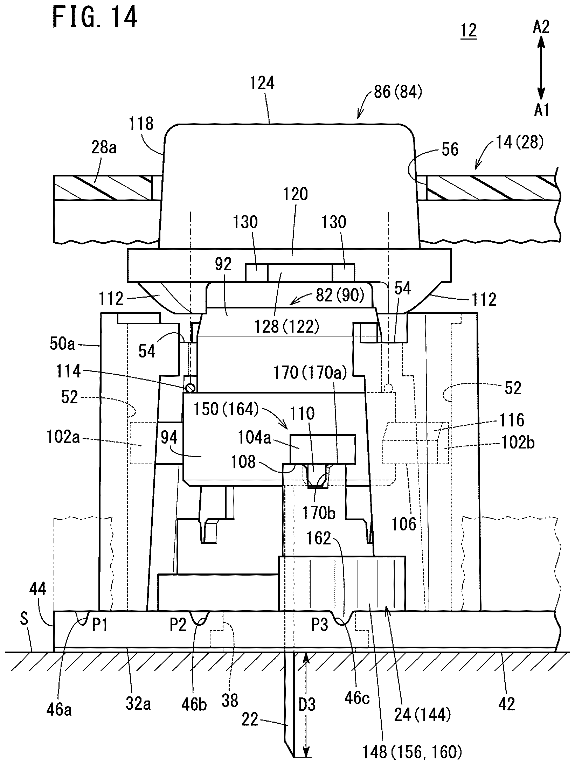

As illustrated in Fig. 14, when the adjustment member 144 is rotated by the operation portion 148 and the protrusion 162 of the operation portion 148 is engaged with the third positioning groove 46c, the abutting portions 104a and 104b of the needle hub 90 face the third adjustment step portions 170, respectively, at a third adjustment position P3. When the needle hub 90 moves in the puncture direction at the third adjustment position P3, the abutting surfaces 108 of the abutting portions 104a and 104b abut on the third abutted portions 170a, respectively. At this time, the puncture depth of the puncture needle 22 with respect to the skin S becomes a third puncture depth D3. The third puncture depth D3 is smaller than the second puncture depth D2, and is, for example, 6 mm. When the abutting portions 104a and 104b of the needle hub 90 abut on the third abutted portions 170a, respectively, the hub-side engagement portions 110 are engaged with the third-step-portion-side engagement portions 170b, respectively.

That is, when the third adjustment position P3 is selected by the adjustment mechanism 24, the puncture depth becomes shallower than the second puncture depth D2 at the second adjustment position P2.

The first to third adjustment step portions 166, 168, and 170 are formed in a stepped shape in which the protruding height with respect to the main body portion 146 changes stepwise in a rotation direction of the adjustment member 144 which is the circumferential direction of the main body portion 146. In the adjustment mechanism 24, the first adjustment step portion 166 is selected to obtain the deepest (largest) puncture depth (first puncture depth D1), the third adjustment step portion 170 is selected to obtain the shallowest (smallest) puncture depth (third puncture depth D3), and the second adjustment step portion 168 is selected to obtain a puncture depth between the first puncture depth D1 and the third puncture depth D3. As the puncture depth, for example, an optimum puncture depth is appropriately selected according to the thickness of subcutaneous fat of the patient.

Note that the movement preventing portion 150 is not limited to the case of including the three first to third adjustment step portions 166, 168, and 170. The quantity is not limited as long as two or more adjustment step portions 164 are provided.

Next, an operation of the liquid medicine administration device 12 including the puncture device 10 will be described. Hereinafter, a case where the adjustment mechanism 24 is set at the first adjustment position P1 and the skin S is punctured by the puncture needle 22 at the first puncture depth D1 as illustrated in Fig. 5 will be described.

First, in the liquid medicine administration device 12 before use (in the initial state) before the puncture is performed as illustrated in Fig. 6, the user (user) performs adjustment to the first adjustment position P1 by the adjustment member 144 of the adjustment mechanism 24 in order to perform the puncture at the first puncture depth D1. The user slides the operation end 156 of the operation portion 148 along the first opening 48 in a direction of approaching the first width-direction wall portion 36a with a finger. At this time, since the operation end 156 has the plurality of grooves 160, slippage of the finger during the operation is prevented, and the operation portion 148 can be reliably moved along the first opening 48.

With the operation of the operation portion 148, the adjustment member 144 rotates around the main body portion 146 along the mounting surface 32b of the housing main body 26. At this time, as viewed along the puncture direction of the needle member 82 illustrated in Fig. 3, the rotation direction of the adjustment member 144 is clockwise. When the protrusion 162 of the operation portion 148 is engaged with the first positioning groove 46a, the first adjustment position P1 at which the abutting portions 104a and 104b of the needle hub 90 and the first adjustment step portions 166 can abut on each other at the time of puncture is obtained (refer to Fig. 6). The abutting portions 104a and 104b partially face the first adjustment step portions 166, respectively, in the axial direction of the needle hub 90.

At this time, in the liquid medicine administration device 12 before use (in the initial state) illustrated in Fig. 6, the abutting portions 104a and 104b of the needle hub 90 are spaced apart from the first adjustment step portions 166 of the adjustment mechanism 24 in the axial direction, and the abutting portions 104a and 104b of the needle hub 90 and the first adjustment step portions 166 are disposed to be offset in the circumferential direction.

Next, the contact surface 32a of the housing 14 is brought into contact with the skin S of the patient (the bonding material 42 is attached to the skin S). As illustrated in Fig. 11, the user pushes the pressing portion 118 (pressing surface 124) of the pusher 86 in the puncture direction (arrow A1 direction), and pushes the pressing portion 118 into the housing 14 through the second hole portion 56. As a result, the pusher 86 moves in the puncture direction (arrow A1 direction) along the pusher guides 134. At this time, the biasing member 114 is compressed in the axial direction toward the needle hub 90 by the movement of the pusher 86 in the puncture direction.

With the movement of the pusher 86, the pusher-side engagement portion 128 presses the inclined surfaces 142 of the pair of lock portions 138 in the puncture direction, and the elastically deformable portions 136 having the lock portions 138 are biased radially outward by the contact with the pusher-side engagement portion 128. When the elastically deformable portions 136 are elastically deformed and the free ends of the elastically deformable portions 136 move radially outward so that the pusher 86 further moves in the puncture direction, the pusher-side engagement portion 128 passes the lock portions 138 and is disposed in the puncture direction with respect to the lock portions 138. At this time, the pusher guides 134 moves between the pair of guide pieces 130, whereby the rotation of the pusher 86 with respect to the pusher guides 134 is prevented. As a result, the pusher 86 moves along the puncture direction.

After the pusher-side engagement portion 128 passes the lock portions 138, the lock portion 138 (the elastically deformable portions 136) are not pressed radially outward any longer, so that the elastically deformable portions 136 return radially inward by elasticity. As a result, the pusher-side engagement portion 128 is engaged with the lock portions 138, and the movement of the pusher-side engagement portion 128 toward the lock portions 138 is prevented. The pressing portion 118 of the pusher 86 is biased in a direction to be spaced apart from the needle hub 90 (direction opposite to the puncture direction) by the biasing member 114, but the pusher 86 is held by the lock portions 138 due to the engagement between the pusher-side engagement portion 128 and the lock portions 138.

At this time, since the pusher 86 and the needle member 82 are not in contact with each other, the needle member 82 does not move from the initial state. That is, when the pusher 86 has moved by a predetermined distance in the puncture direction in a state where the needle member 82 does not move, the lock portions 138 can prevent the movement of the pusher 86 in the direction opposite to the puncture direction by engaging with the pusher-side engagement portion 128 of the pusher 86 (the protruding portion 122). As a result, the biasing force of the biasing member 114 in the puncture direction (arrow A1 direction) is stored.

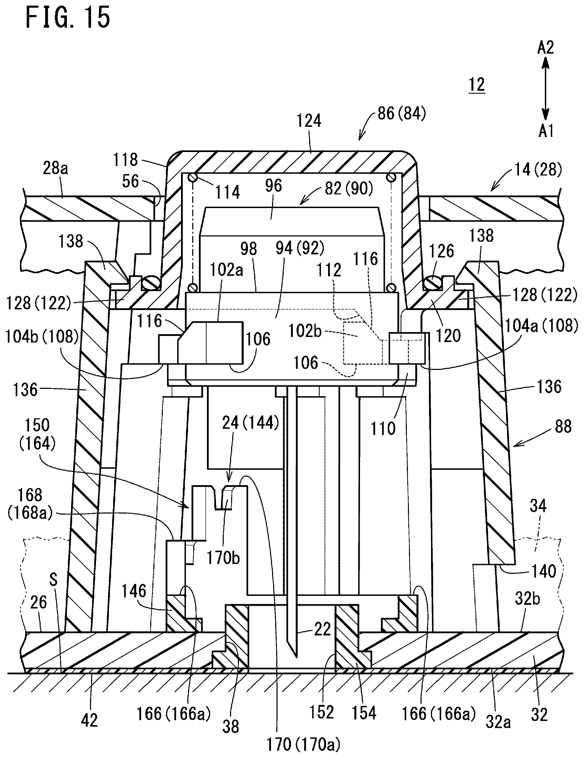

As illustrated in Fig. 15, when the pusher 86 is further pushed in the puncture direction, the first inclined portions 112 of the pusher 86 press the second inclined portions 116 of the needle hub 90 in the puncture direction. At this time, as illustrated in Fig. 5, the guided portions 102a and 102b having the second inclined portions 116 are prevented from moving in the puncture direction as the locking surfaces 106 abut on the locking portions 54 of the guide columns 50a and 50b, respectively, of the housing 14. A position of the needle member 82 in the rotation direction with respect to the housing 14 in the initial state illustrated in Fig. 5 is referred to as a "first position".

When the second inclined portions 116 of the needle hub 90 are pushed in the puncture direction (arrow A1 direction) by the first inclined portions 112 of the pusher 86, the pair of guided portions 102a and 102b receives a force counterclockwise when viewed in the puncture direction due to the inclination of the first inclined portions 112 and the second inclined portions 116 (refer to Fig. 5). As a result, the needle hub 90 does not move in the puncture direction and starts to rotate in the counterclockwise direction from the first position, around the axis of the needle hub 90. The locking surfaces 106 of the needle hub 90 move along the locking portions 54 of the guide columns 50a and 50b.

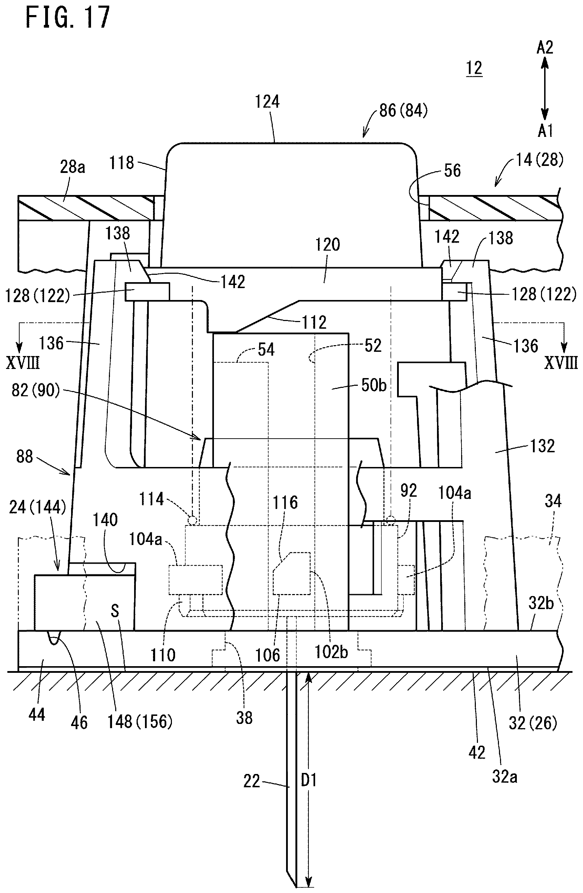

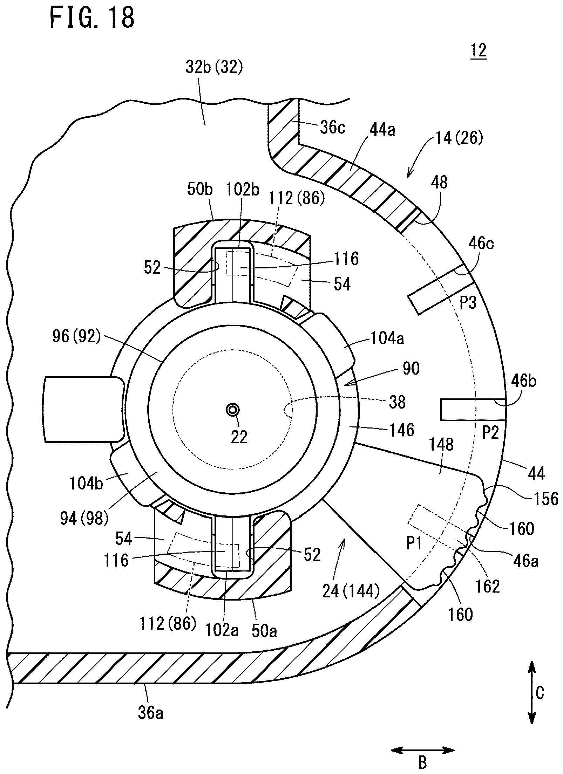

As illustrated in Fig. 18, when the needle hub 90 rotates and reaches a second position where the pair of guided portions 102a and 102b face proximal ends of the guide grooves 52, respectively, and the locking surfaces 106 are separated from engagement surfaces, the needle hub 90 moves in the puncture direction (arrow A1 direction) by the biasing force of the biasing member 114. At this time, as illustrated in Fig. 17, the guided portions 102a and 102b are inserted into the guide grooves 52, and the guided portions 102a and 102b are guided in the puncture direction along the guide grooves 52, respectively, whereby the needle hub 90 moves. At this time, the abutting portions 104a and 104b of the needle hub 90 face the first adjustment step portions 166 of the adjustment member 144, respectively, in the puncture direction.

The needle hub 90 moves in the puncture direction, and the puncture needle 22 protrudes from the contact surface 32a toward the skin S through the second hole portion 56. The puncture needle 22 punctures the skin S of the patient. As the needle member 82 moves in the puncture direction, the tube 100 is elastically deformed. At this time, the abutting portions 104a and 104b of the needle member 82 abut on the first abutted portion 166a of the first adjustment step portion 166, so that further movement of the needle member 82 in the puncture direction is prevented. The puncture depth by the puncture needle 22 becomes the first puncture depth D1 by the contact between the abutting portions 104a and 104b and the first adjustment step portions 166.

As illustrated in Fig. 12, the hub-side engagement portions 110 of the abutting portions 104a and 104b are engaged with the first-step-portion-side engagement portions 166b of the first adjustment step portions 166, respectively, whereby the relative rotation between the needle member 82 and the adjustment member 144 is prevented. As a result, the abutment state between the abutting portions 104a and 104b of the needle member 82 and the first adjustment step portions 166 is suitably maintained. The engagement between the hub-side engagement portions 110 and the first-step-portion-side engagement portions 166b prevents the adjustment member 144 from rotating with respect to the needle member 82 and prevents the abutting portions 104a and 104b from moving to other adjustment step portions to change the puncture depth.

After the puncture of the puncture needle 22 to the skin S as illustrated in Fig. 17 is completed, by turning on a power supply (not illustrated) of the liquid medicine administration device 12, power is supplied from the power supply unit 74 illustrated in Fig. 1 to the motor 68, and the motor 68 is driven. As the motor 68 is driven, each of the drive gear 76, the intermediate gear 80, and the driven gear 78 rotates, the shaft 72 rotates, so that the rod 62 moves in the distal direction in the barrel 58. When the gasket 60 is pushed in the distal direction by the rod 62, the liquid medicine M in the medicine chamber 66 is delivered from the nozzle 64 to the tube 100. The liquid medicine M is supplied to the communication path of the needle hub 90, and is subcutaneously administered to the patient through the puncture needle 22.

As illustrated in Fig. 13, in a case where the puncture depth of the puncture needle 22 is set to the second puncture depth D2 by the adjustment member 144, the user operates the operation portion 148 to move the operation portion 148 to the second adjustment position P2 in the liquid medicine administration device 12 before use (in the initial state). The operation of the operation portion 148 makes the adjustment member 144 rotate counterclockwise along the attachment portion 44. The protrusion 162 of the operation portion 148 is engaged with the second positioning groove 46b and is positioned. The second adjustment step portions 168 are disposed at positions capable of abutting on the abutting portions 104a and 104b of the needle member 82. When the pusher 86 is pushed and the needle member 82 is moved in the puncture direction by the biasing member 114, the abutting portions 104a and 104b of the needle member 82 abut on the second abutted portions 168a of the second adjustment step portions 168 and are locked. As a result, a movement amount of the needle member 82 in the puncture direction is controlled, and the puncture needle 22 punctures the skin S at the second puncture depth D2.

As illustrated in Fig. 14, in a case where the puncture depth of the puncture needle 22 is set to the third puncture depth D3 by the adjustment member 144, the user operates the operation portion 148 to move the operation portion 148 to the third adjustment position P3 in the liquid medicine administration device 12 before use (in the initial state). The operation of the operation portion 148 makes the adjustment member 144 rotate counterclockwise along the attachment portion 44. The protrusion 162 of the operation portion 148 is engaged with the third positioning groove 46c and is positioned. The third adjustment step portions 170 are disposed at positions capable of abutting on the abutting portions 104a and 104b of the needle member 82. When the pusher 86 is pushed and the needle member 82 is moved in the puncture direction by the biasing member 114, the abutting portions 104a and 104b of the needle member 82 abut on the third abutted portions 170a of the third adjustment step portions 170 and are locked. As a result, the movement amount of the needle member 82 in the puncture direction is controlled, and the puncture needle 22 punctures the skin S at the third puncture depth D3.

That is, when the puncture needle 22 moves toward the skin S to puncture the skin S by the puncture needle 22, it is possible to control the movement amount of the needle member 82 in the puncture direction and adjust the puncture depth of the puncture needle 22 with respect to the skin S by adjusting positions of the first to third adjustment step portions 166, 168, and 170 with respect to the housing 14 and making each of the abutting portions 104a and 104b of the needle hub 90 abut on one of the first to third adjustment step portions 166, 168, and 170.

As described above, in the embodiment of the present invention, the adjustment mechanism 24 capable of adjusting the puncture depth of the skin S by the puncture needle 22 is provided, and the user operates the adjustment mechanism 24 to set the positions of the first to third adjustment step portions 166, 168, and 170 with respect to the housing 14 before puncturing the skin S with the puncture needle 22, so that each of the abutting portions 104a and 104b of the needle member 82 can abut on one adjustment step portion of the first to third adjustment step portions 166, 168, and 170 according to the positions of the first to third adjustment step portions 166, 168, and 170 with respect to the housing 14, and the puncture depth of the puncture needle 22 with respect to the skin S can be adjusted. As a result, the adjustment mechanism 24 can easily select the puncture depth of the puncture needle 22 with respect to the skin S of a living body and perform the puncture of the skin S at a desired depth.

Since the adjustment mechanism 24 is disposed so as to be displaceable along the bottom wall 32 (mounting surface 32b) of the housing main body 26 disposed perpendicular to the axis of the puncture needle 22, when the puncture of the skin S is performed by the puncture needle 22, any one of the plurality of first to third adjustment step portions 166, 168, and 170 and each of the abutting portions 104a and 104b of the needle member 82 can be disposed to face each other by moving the adjustment mechanism 24 along the bottom wall 32 before puncturing the skin S with the puncture needle 22.

Since the adjustment mechanism 24 is rotatably disposed with respect to the housing main body 26, when the skin S is punctured by the puncture needle 22, any one of the plurality of first to third adjustment step portions 166, 168, and 170 and each of the abutting portions 104a and 104b of the needle member 82 can be disposed at positions facing each other by rotating the adjustment mechanism 24 before puncturing the skin S with the puncture needle 22.

Since the adjustment mechanism 24 is disposed so as to be rotatable with respect to the housing 14 around the axis of the puncture needle 22, the needle hub 90 having the puncture needle 22 and the first to third adjustment step portions 166, 168, and 170 of the adjustment mechanism 24 can be coaxially disposed. As a result, each of the abutting portions 104a and 104b of the needle hub 90 can reliably abut on one of the first to third adjustment step portions 166, 168, and 170 at the time of puncturing the skin S with the puncture needle 22.

When the user operates the operation portion 148 of the adjustment member 144, the adjustment mechanism 24 can be easily moved with respect to the housing 14, and any one of the first to third adjustment step portions 166, 168, and 170 can be disposed to face each of the abutting portions 104a and 104b of the needle member 82.

When one of the first to third adjustment step portions 166, 168, and 170 and each of the abutting portions 104a and 104b of the needle hub 90 abut on each other, any of the first to third-step-portion-side engagement portions 166b, 168b, and 170b and the hub-side engagement portion 110 are engaged, so that a position of the adjustment mechanism 24 is maintained and a desired puncture depth is suitably maintained. Therefore, among the first to third adjustment step portions 166, 168, and 170, other adjustment step portions that do not abut on the abutting portions 104a and 104b are prevented from erroneously abutting on the abutting portions 104a and 104b to change the puncture depth of the puncture needle 22.

Since the biasing member 114 that biases the needle member 82 in the puncture direction is provided, the puncture needle 22 of the needle member 82 can be caused to protrude from the contact surface 32a of the housing 14 by the biasing force of the biasing member 114, and the skin S can be reliably punctured by the puncture needle 22.

When the user pushes the pusher 86 toward the housing 14, the pusher 86 is guided in the puncture direction by the pusher guides 134, so that the pusher 86 can be stably moved in the puncture direction.

Since the pusher 86 is pushed in the puncture direction and the pusher-side engagement portion 128 of the pusher 86 is locked by the lock portions 138 of the guide member 88, the movement of the pusher 86 in the direction opposite to the puncture direction is effectively prevented, and the biasing force of the biasing member 114 for puncture can be effectively secured. As a result, the needle member 82 is biased in the puncture direction by the biasing force of the biasing member 114 so that reliable puncture can be performed.

Note that the present invention is not limited to the above disclosure, and various configurations can be adopted without departing from the gist of the present invention.

## Claims

1. A puncture device comprising:
a housing having a contact surface that comes into contact with skin of a living body;
a needle member that comprises a puncture needle and is movable relative to the housing along an axis of the puncture needle, the puncture needle being capable of protruding from the contact surface toward the skin; and
an adjustment mechanism that is displaceable with respect to the housing and adjusts a puncture depth of the puncture needle with respect to the skin,
wherein
the adjustment mechanism comprises a movement preventing portion that abuts on an abutting portion of the needle member and prevents movement in a puncture direction when the puncture needle moves toward the skin to perform puncture of the skin,
the movement preventing portion comprises a plurality of adjustment step portions having different distances from the abutting portion of the needle member in the puncture direction in a state before the puncture of the skin by the puncture needle is performed, and
the abutting portion abuts on one of the plurality of adjustment step portions according to a position of the adjustment mechanism with respect to the housing to adjust a depth of the puncture needle with respect to the skin when the puncture needle moves toward the skin to perform the puncture of the skin.

2. The puncture device according to claim 1, wherein
the adjustment mechanism is displaceable along a plane perpendicular to the axis with respect to the housing.

3. The puncture device according to claim 2, wherein
the adjustment mechanism is rotatable with respect to the housing.

4. The puncture device according to claim 3, wherein
the adjustment mechanism is rotatable around the axis of the puncture needle with respect to the housing.

5. The puncture device according to any one of claims 1 to 4, wherein
the adjustment mechanism comprises an operation portion that is movable along a direction in which the plurality of adjustment step portions are arranged in parallel and protrudes to an outside of the housing.

6. The puncture device according to any one of claims 1 to 4, wherein
each of the plurality of adjustment step portions comprises a step-portion-side engagement portion comprising a recess or a projection, and
the abutting portion comprises a hub-side engagement portion comprising a projection or a recess to be engaged with the recess or the projection of the step-portion-side engagement portion.

7. The puncture device according to any one of claims 1 to 4, comprising
a biasing member that is provided in the housing and biases the needle member toward the skin.

8. The puncture device according to claim 7, comprising
a puncture mechanism that causes the needle member to protrude toward the skin with respect to the contact surface of the housing,
wherein the puncture mechanism comprises a pusher that has a first inclined portion inclined with respect to the puncture direction of the puncture needle and is movable in the puncture direction with respect to the housing,
the biasing member, and
a second inclined portion that is provided in the needle member, is inclined with respect to the puncture direction, and abuts on the first inclined portion,
when the pusher moves in the puncture direction, the needle member rotates from a first position to a second position around an axis along the puncture direction, via the first inclined portion and the second inclined portion, and
the needle member reaching the second position moves in the puncture direction by a biasing force of the biasing member.

9. The puncture device according to claim 8, comprising
a guide member having a pusher guide that guides the pusher along the puncture direction.

10. The puncture device according to claim 9, comprising
a lock portion that engages with the pusher and prevents the pusher from moving in a direction opposite to the puncture direction against the biasing force of the biasing member when the pusher has moved in the puncture direction by a predetermined distance in a range in which the needle member is not rotated.

11. The puncture device according to claim 10, wherein
the guide member comprises the lock portion.

12. A liquid medicine administration device comprising:
the puncture device according to any one of claims 1 to 4; and
a prefilled syringe that is filled with a liquid medicine, is connected to the needle member of the puncture device, and delivers the liquid medicine to the puncture needle.
